# EUROPEAN PATENT APPLICATION

(11) **EP 3 868 216 A1**
(43) Date of publication of application: **25.08.2021**
(21) Application number: 19874159.7
(22) Date of filing: 16.10.2019
(51) Int. Cl.: A23L 33/10, A61K 31/194, A61P 1/00, A61P 3/00, A61P 25/22, A61P 39/00

(54) **AGENT FOR SUPPRESSING ALCOHOL SICKNESS OR HANGOVER CAUSED BY ALCOHOLIC BEVERAGE INTAKE**

(30) Priority: 16.10.2018 JP 2018195043
(71) Applicant: Nippon Chemiphar Co., Ltd., Tokyo 101-0032 (JP)
(72) Inventor: KAWAGUCHI Kazuhiko, Tokyo 101-0032 (JP); YAMASAKI Satomi, Tokyo 101-0032 (JP)
(74) Representative: Beckmann, Claus
(86) International application number: PCT/JP2019/040653
(87) International publication number: WO 2020/080398

(57) **Abstract**

The present invention provides a food composition or pharmaceutical composition which includes, as an active ingredient, citric acid or a salt thereof. Ingestion or administration of the food composition or pharmaceutical composition leads to suppression of alcohol sickness or hangover due to alcoholic beverage ingestion; suppression of an increase in blood alcohol level, an increase in blood acetaldehyde level, or an increase in blood acetic acid level due to alcoholic beverage ingestion; suppression of a decrease in blood pyruvic acid level due to alcoholic beverage ingestion; suppression of an abnormal blood lactic acid/pyruvic acid ratio due to alcoholic beverage ingestion; suppression of an increase in blood alcohol level immediately after alcohol ingestion; suppression of alcohol absorption; promotion of alcohol metabolism; or an increase in blood ornithine level, blood serotonin level, blood taurine level, blood cystine level, or blood nicotine amide level.

## Description

### Technical Field

The present invention relates to a food composition or pharmaceutical composition for suppressing alcohol sickness or hangover due to alcoholic beverage ingestion; suppressing an increase in blood alcohol level, an increase in blood acetaldehyde level, or an increase in blood acetic acid level due to alcoholic beverage ingestion; suppressing a decrease in blood pyruvic acid level due to alcoholic beverage ingestion; suppressing an abnormal blood lactic acid/pyruvic acid ratio due to alcoholic beverage ingestion; suppressing an increase in blood alcohol level immediately after alcohol ingestion; suppressing alcohol absorption; promoting alcohol metabolism; or increasing a blood ornithine level, a blood serotonin level, a blood taurine level, a blood cystine level, or a blood nicotine amide level, the food composition or pharmaceutical composition including, as an active ingredient, citric acid or a salt thereof.

The present application claims priority based on Japanese Patent Application No. 2018-195043 filed in Japan on October 16, 2018, and the contents are incorporated herein by reference.

### Background Art

Alcohol (particularly ethanol) is known to affect the physiological and psychological states, and the ingestion of relatively small amounts of alcoholic beverages may also contribute to the improvement of the quality of sleep, for example, it allows people to be in a cheerful mood or decreases the time to onset of sleep. However, the ingestion of excessive alcoholic beverages is known to cause so-called or alcohol sickness or hangover condition accompanied by nausea, vomiting, headache, or dizziness. Ingested alcohol is metabolized to acetic acid via acetaldehyde in vivo and further decomposed into carbon dioxide and water, but it is understood that acetaldehyde produced by the metabolism of alcohol is deeply involved in alcohol sickness or hangover. Aldehyde dehydrogenase 2 is an enzyme that metabolizes acetaldehyde to acetic acid in vivo. It is known that the percentage of Japanese who do not have normal aldehyde dehydrogenase 2 is higher than that of Westerners. Individuals who do not have aldehyde dehydrogenase 2 are classified as "flushers" and are prone to cause flushing reactions (such as flushing, palpitation, nausea, and hypotension) when they drink small amounts of alcohol.

Ornithine is one of the amino acids and is known as a substance constituting the ornithine cycle that converts harmful ammonia into urea in the living body. Ornithine is also expected to be effective in suppressing alcohol sickness or hangover due to alcoholic beverage ingestion. Further, it has been reported that ornithine orally administered causes a decrease in salivary cortisol and an improvement in feeling of fatigue the next morning after flushers drank alcohol (Non Patent Literature 1).

It is known that cystine (also referred to as "3,3'-dithiobis(2-aminopropionic acid)") is one of the amino acids, and is contained in a large amount as a constituent of keratin that constitutes hair or nails. Further, cystine is known to have the effect of promoting the metabolism of acetaldehyde in vivo.

Nicotinamide (also referred to as "niacin or vitamin B₃") is known as a constituent of oxidoreductase coenzymes: nicotinamide adenine dinucleotide (abbreviated as: NAD) and nicotinamide adenine dinucleotide phosphate (abbreviated as: NADP) in vivo. These coenzymes function as electron acceptors or hydrogen donors for redox reactions in vivo. For example, NAD also functions as a coenzyme for alcohol dehydrogenase and acetaldehyde dehydrogenase, which are alcohol-metabolizing enzymes. When alcohol is metabolized to acetaldehyde, the oxidized form NAD⁺ is reduced to the reduced form NADH. When acetaldehyde is metabolized to acetic acid, the oxidized form NAD⁺ is reduced to the reduced form NADH. Nicotinamide is biosynthesized from tryptophan in vivo. Tryptophan is also known to be a biosynthetic raw material for serotonin and melatonin. Serotonin is synthesized in vivo from tryptophan through 5-hydroxytryptophan. It is known that most of serotonin is distributed in the mucous membrane of the digestive tract and acts on the peristaltic movement of the intestine, but it also exists as a neurotransmitter in the brain, and is responsible for physiological functions such as biological rhythm, sleep or thermoregulation, and psychological functions such as mental stabilization. Further, it is known that serotonin secreted during the day is converted to melatonin from evening to midnight, and such melatonin promotes falling asleep. When nicotine amide is consumed by alcohol metabolism, tryptophan is consumed for nicotinamide biosynthesis to supplement it, and the proportion of tryptophan used for serotonin or melatonin biosynthesis is decreased. As a result, a shortage of serotonin or melatonin may adversely affect the quality of sleep. Therefore, the ingestion of nicotinamide is expected to contribute to the promotion of alcohol and acetaldehyde metabolism and the suppression of deterioration of quality of sleep during alcohol metabolism.

Taurine (also referred to as "2-aminoethanesulfonic acid") is one of the sulfur-containing amino acid-like substances existing in the living body, and is known to be involved in the maintenance of homeostasis in vivo and have a physical fatigue recovery effect. Further, taurine is also known to promote the metabolism of acetaldehyde in vivo.

It is known that pyruvic acid is reduced in vivo by lactate dehydrogenase (LDH) and converted to lactic acid. Such a conversion reaction is a reversible reaction. Usually, the blood lactic acid level/pyruvic acid level ratio is kept approximately constant. In the conversion reaction between pyruvic acid and lactic acid, NAD functions as a coenzyme. That is, in the conversion reaction from pyruvic acid to lactic acid, the reduced form NADH is oxidized to the oxidized form NAD⁺. In the conversion reaction from lactic acid to pyruvic acid, the oxidized form NAD⁺ is reduced to the reduced form NADH. Alcohol metabolism consumes the oxidized form NAD⁺, so ingestion of alcohol results in shortage of the oxidized form NAD⁺ required for the conversion reaction of lactic acid to pyruvic acid. Therefore, when alcohol is ingested, the conversion reaction from lactic acid to pyruvic acid does not progresses, the blood pyruvic acid level decreases, and the lactic acid/pyruvic acid ratio increases. The blood pyruvic acid level is increased and the lactic acid/pyruvic acid ratio is made closer to normal, so that the oxidized form NAD⁺ can be supplied. Thus, the alcohol metabolism can be promoted (Patent Literature 1).

Incidentally, citric acid is known to be useful as an additive such as a stabilizer, a buffer, or a flavoring agent in the field of medical drugs or foods.

It is known that the administration of a salt of citric acid, particularly an alkali metal salt (hereinafter, these may be collectively referred to as "alkalizing agent") to patients with hyperuricemia or gout is useful in suppressing the formation of urinary stones in these patients. Further, it is known that since the alkalizing agent has an effect of making the body fluid of a patient alkaline, it is effective in improving the acidosis of the patient, particularly the metabolic acidosis such as renal tubular acidosis.

However, it is not known that citric acid or a salt thereof has an effect of suppressing an increase in blood alcohol level, an increase in blood acetaldehyde level, or an increase in blood acetic acid level; an effect of suppressing a decrease in blood pyruvic acid level due to alcoholic beverage ingestion; an effect of suppressing an abnormal blood lactic acid/pyruvic acid ratio due to alcoholic beverage ingestion; an effect of suppressing an increase in blood alcohol level immediately after alcohol ingestion; an effect of suppressing alcohol absorption; an effect of promoting alcohol metabolism; or an effect of increasing a blood ornithine level, a blood serotonin level, a blood taurine level, a blood cystine level, or a blood nicotine amide level, in mammals (particularly humans). Furthermore, citric acid or a salt thereof is not known to be useful in suppressing alcohol sickness or hangover due to alcoholic beverage ingestion in mammals (particularly humans).

### Citation List

### Patent Literature

Patent Literature 1: JP 2019-43861 A

### Non Patent Literature

Non Patent Literature 1: Kokubo et al., BioPsychoSocial Medicine 2013, 7: 6

### Summary of Invention

### Technical Problem

One of the objects of the present invention is to provide a food composition or pharmaceutical composition for suppressing alcohol sickness or hangover due to alcoholic beverage ingestion in mammals (particularly humans). Another object of the present invention is to provide a food composition or pharmaceutical composition for suppressing an increase in blood alcohol level, an increase in blood acetaldehyde level, or an increase in blood acetic acid level due to alcoholic beverage ingestion in mammals (particularly humans). Another object of the present invention is to provide a food composition or pharmaceutical composition for suppressing a decrease in blood pyruvic acid level due to alcoholic beverage ingestion in mammals (particularly humans). Another object of the present invention is to provide a food composition or pharmaceutical composition for suppressing an abnormal blood lactic acid/pyruvic acid ratio due to alcoholic beverage ingestion in mammals (particularly humans). Another object of the present invention is to provide a food composition or pharmaceutical composition for suppressing an increase in blood alcohol level immediately after alcohol ingestion in mammals (particularly humans). Another object of the present invention is to provide a food composition or pharmaceutical composition for suppressing alcohol absorption in mammals (particularly humans). Another object of the present invention is to provide a food composition or pharmaceutical composition for promoting alcohol metabolism in mammals (particularly humans). Further, another object of the present invention is to provide a food composition or pharmaceutical composition for increasing a blood ornithine level, a blood serotonin level, a blood taurine level, a blood cystine level, or a blood nicotine amide level in mammals (particularly humans).

### Solution to Problem

As a result of diligent studies to achieve the above objects, the present inventors have found that citric acid or a salt thereof has an effect of suppressing an increase in blood alcohol level, an increase in blood acetaldehyde level, or an increase in blood acetic acid level due to alcoholic beverage ingestion in mammals (particularly humans); an effect of suppressing a decrease in blood pyruvic acid level due to alcoholic beverage ingestion; an effect of suppressing an abnormal blood lactic acid/pyruvic acid ratio due to alcoholic beverage ingestion; an effect of suppressing an increase in blood alcohol level immediately after alcohol ingestion; an effect of suppressing alcohol absorption; an effect of promoting alcohol metabolism; or an effect of increasing a blood ornithine level, a blood serotonin level, a blood taurine level, a blood cystine level, or a blood nicotine amide level, and the citric acid or the salt thereof is useful in suppressing alcohol sickness or hangover due to alcoholic beverage ingestion in mammals (particularly humans), and they have completed the present invention.

The present invention has the following aspects:
(1) A food composition or pharmaceutical composition for suppressing alcohol sickness or hangover due to alcoholic beverage ingestion in mammals (particularly humans), including, as an active ingredient, citric acid or a salt thereof.
(2) A food composition or pharmaceutical composition for suppressing an increase in blood alcohol level, an increase in blood acetaldehyde level, or an increase in blood acetic acid level due to alcoholic beverage ingestion in mammals (particularly humans), including, as an active ingredient, citric acid or a salt thereof.
(3) A food composition or pharmaceutical composition for suppressing a decrease in blood pyruvic acid level due to alcoholic beverage ingestion in mammals (particularly humans), including, as an active ingredient, citric acid or a salt thereof.
(4) A food composition or pharmaceutical composition for suppressing an abnormal blood lactic acid/pyruvic acid ratio due to alcoholic beverage ingestion in mammals (particularly humans), including, as an active ingredient, citric acid or a salt thereof.
(5) A food composition or pharmaceutical composition for suppressing an increase in blood alcohol level immediately after alcohol ingestion in mammals, including, as an active ingredient, citric acid or a salt thereof.
(6) A food composition or pharmaceutical composition for suppressing alcohol absorption in mammals, including, as an active ingredient, citric acid or a salt thereof.
(7) A food composition or pharmaceutical composition for promoting alcohol metabolism in mammals, including, as an active ingredient, citric acid or a salt thereof.
(8) The food composition or pharmaceutical composition according to any one of (1) to (7), wherein the active ingredient is an alkali metal salt of citric acid.
(9) The food composition or pharmaceutical composition according to any one of (1) to (8), wherein the alkali metal salt of citric acid includes a mixture of sodium citrate or a hydrate thereof and potassium citrate or a hydrate thereof.
(10) The food composition or pharmaceutical composition according to any one of (1) to (9) including:
   sodium citrate or a hydrate thereof; potassium citrate or a hydrate thereof; and anhydrous citric acid.
(11) The food composition or pharmaceutical composition according to any one of (1) to (10), wherein the food composition or pharmaceutical composition is a food composition whose package, container, or instruction indicates an effect of suppressing alcohol sickness or hangover due to alcoholic beverage ingestion; an effect of suppressing an increase in blood alcohol level, an increase in blood acetaldehyde level, or an increase in blood acetic acid level due to alcoholic beverage ingestion; an effect of suppressing a decrease in blood pyruvic acid level due to alcoholic beverage ingestion; an effect of suppressing an abnormal blood lactic acid/pyruvic acid ratio due to alcoholic beverage ingestion; an effect of suppressing an increase in blood alcohol level immediately after alcohol ingestion; an effect of suppressing alcohol absorption;
   and/or an effect of promoting alcohol metabolism.
(12) A food composition or pharmaceutical composition for increasing a blood ornithine level, a blood serotonin level, a blood taurine level, a blood cystine level, or a blood nicotine amide level in mammals (particularly humans), including, as an active ingredient, citric acid or a salt thereof.
(13) The food composition or pharmaceutical composition according to (12), wherein the active ingredient is an alkali metal salt of citric acid.
(14) The food composition or pharmaceutical composition according to any one of (1) to (13), wherein the active ingredient is anhydrous citric acid, sodium citrate or a hydrate thereof, and potassium citrate or a hydrate thereof, a molar ratio of the anhydrous citric acid: the sodium citrate or the hydrate thereof: the potassium citrate or the hydrate thereof is in a range of 1 : 1.7-2.3 : 1.7-2.3, and the food composition or pharmaceutical composition is administered or ingested before alcohol ingestion.
(15) The food composition or pharmaceutical composition according to any one of (1) to (14), wherein a dosage form is a tablet.

### Advantageous Effects of Invention

Ingestion or administration of the food composition or pharmaceutical composition including citric acid or a salt thereof as an active ingredient, which is provided by the present invention, leads to suppression of alcohol sickness or hangover due to alcoholic beverage ingestion; suppression of an increase in blood alcohol level, an increase in blood acetaldehyde level, or an increase in blood acetic acid level due to alcoholic beverage ingestion; suppression of a decrease in blood pyruvic acid level due to alcoholic beverage ingestion; suppression of an abnormal blood lactic acid/pyruvic acid ratio due to alcoholic beverage ingestion; suppression of an increase in blood alcohol level immediately after alcohol ingestion; suppression of alcohol absorption; promotion of alcohol metabolism; or an increase in blood ornithine level, blood serotonin level, blood taurine level, blood cystine level, or blood nicotine amide level, in mammals, particularly humans.

### Brief Description of Drawings

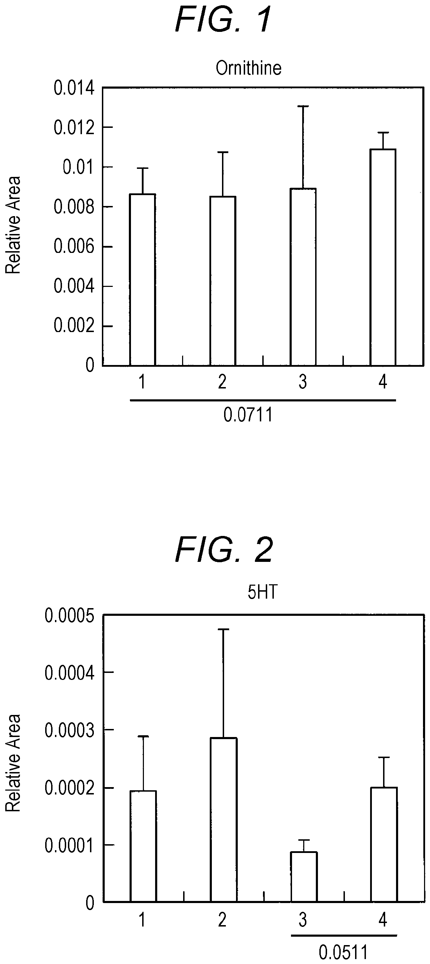

Fig. 1 is a graph illustrating changes in plasma ornithine level when a combination drug of potassium citrate and sodium citrate hydrate has been administered to miniature pigs with renal failure. The graphs 1, 2, 3, and 4 on the horizontal axis show the results before administration, after the first week of administration, after the second week of discontinuation of administration, and after the third week of administration, respectively, and the vertical axis shows the relative area value of each test substance amount (the same applies to Figs. 2 to 5).
Fig. 2 is a graph illustrating changes in plasma serotonin level when a combination drug of potassium citrate and sodium citrate hydrate has been administered to miniature pigs with renal failure.

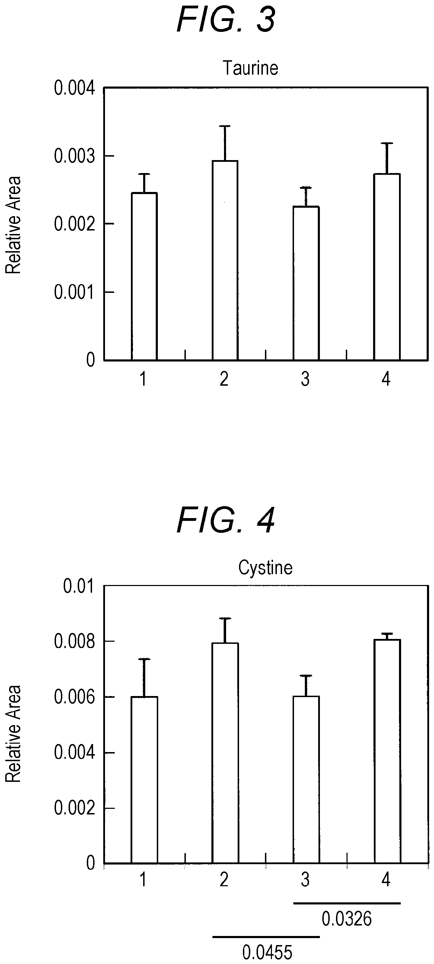

Fig. 3 is a graph illustrating changes in plasma taurine level when a combination drug of potassium citrate and sodium citrate hydrate has been administered to miniature pigs with renal failure.
Fig. 4 is a graph illustrating changes in plasma cystine level when a combination drug of potassium citrate and sodium citrate hydrate has been administered to miniature pigs with renal failure.

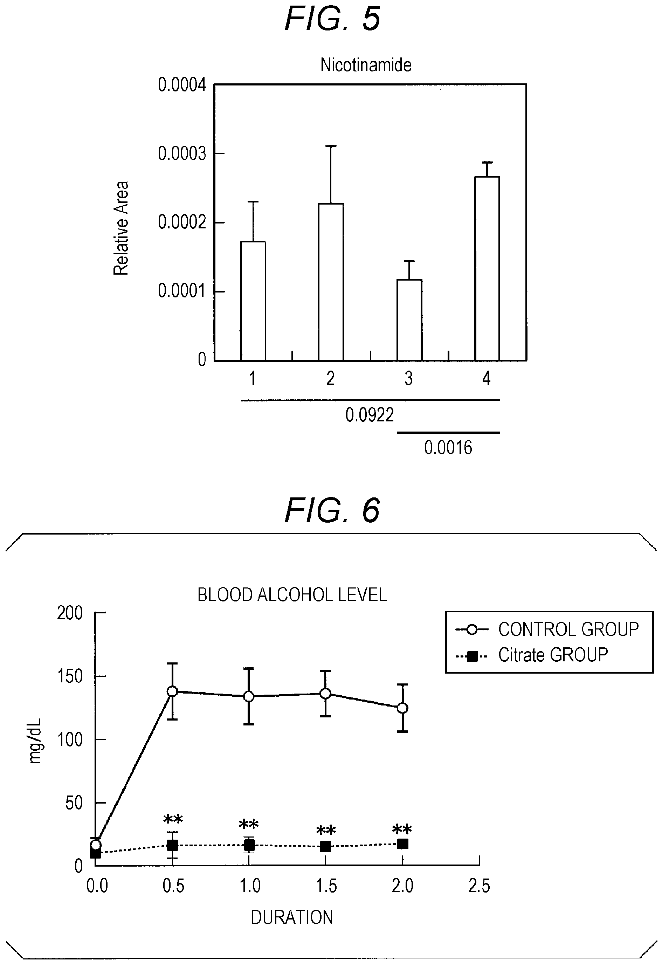

Fig. 5 is a graph illustrating changes in plasma nicotinamide level when a combination drug of potassium citrate and sodium citrate hydrate has been administered to miniature pigs with renal failure.
Fig. 6 is a graph illustrating changes in blood alcohol level when an ethanol solution has been administered respectively to a citrate group after administration of a combination drug of potassium citrate and sodium citrate hydrate to SD male rats (8 weeks old) and a control group after administration of purified water to SD male rats (8 weeks old).

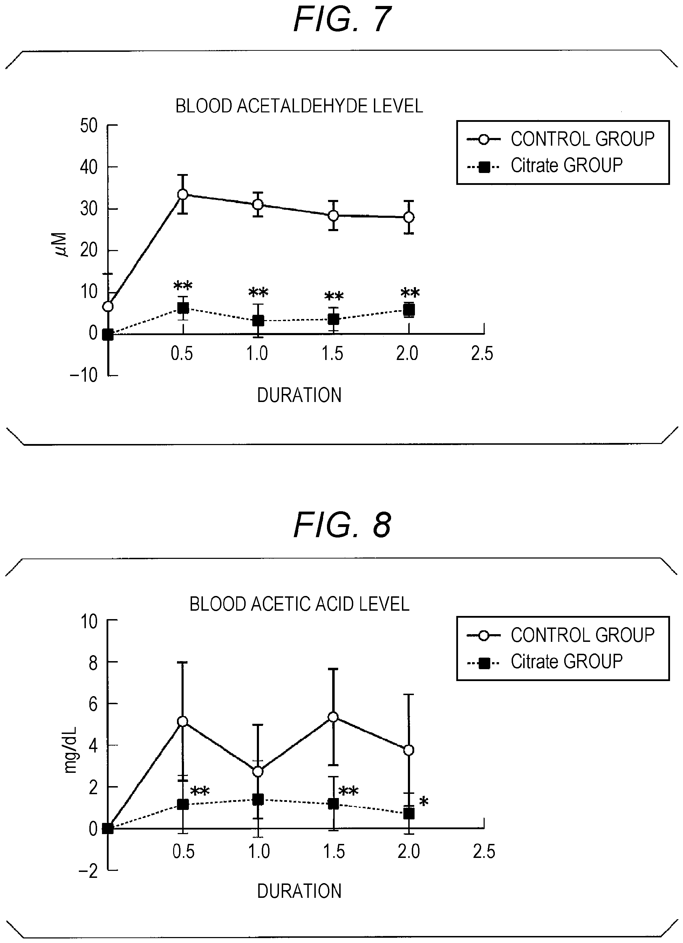

Fig. 7 is a graph illustrating changes in blood acetaldehyde level when an ethanol solution has been administered respectively to a citrate group after administration of a combination drug of potassium citrate and sodium citrate hydrate to SD male rats (8 weeks old) and a control group after administration of purified water to SD male rats (8 weeks old).
Fig. 8 is a graph illustrating changes in blood acetic acid level when an ethanol solution has been administered respectively to a citrate group after administration of a combination drug of potassium citrate and sodium citrate hydrate to SD male rats (8 weeks old) and a control group after administration of purified water to SD male rats (8 weeks old).

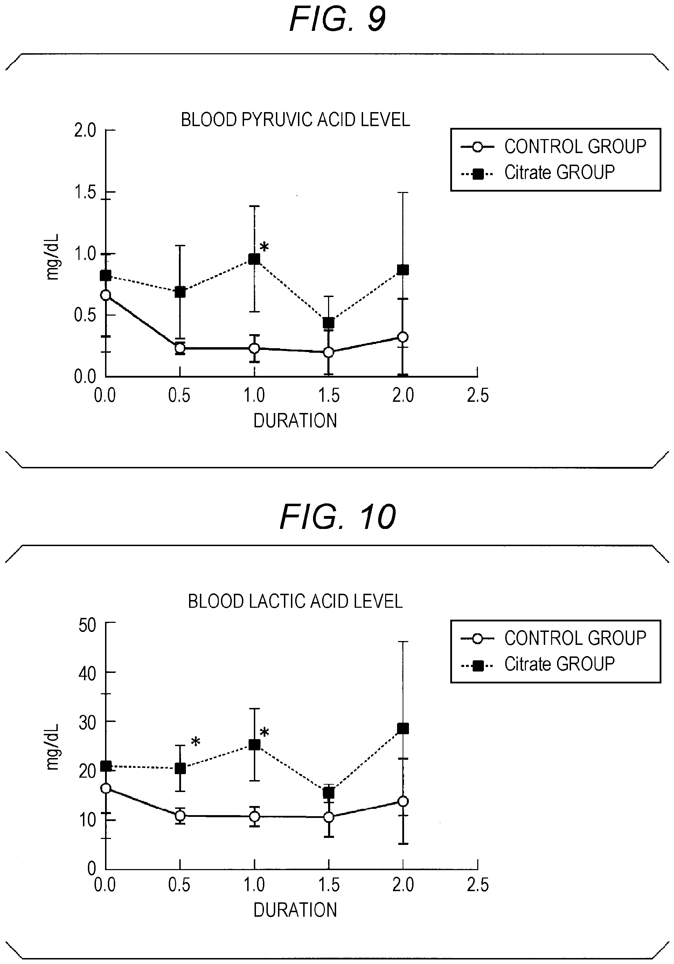

Fig. 9 is a graph illustrating changes in blood pyruvic acid level when an ethanol solution has been administered respectively to a citrate group after administration of a combination drug of potassium citrate and sodium citrate hydrate to SD male rats (8 weeks old) and a control group after administration of purified water to SD male rats (8 weeks old).
Fig. 10 is a graph illustrating changes in blood lactic acid level when an ethanol solution has been administered respectively to a citrate group after administration of a combination drug of potassium citrate and sodium citrate hydrate to SD male rats (8 weeks old) and a control group after administration of purified water to SD male rats (8 weeks old).

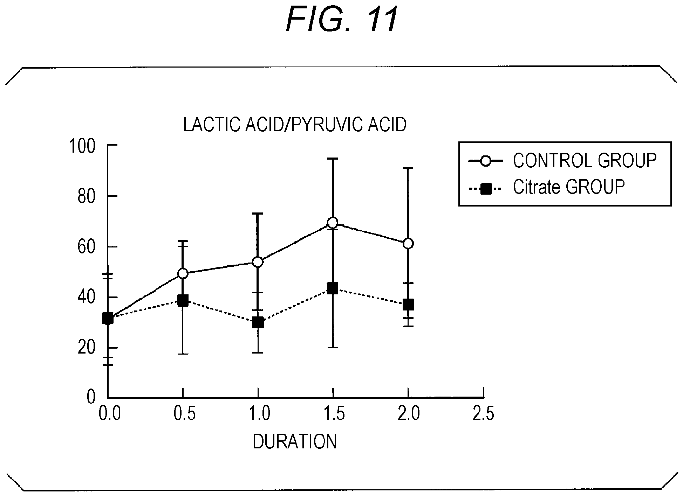

Fig. 11 is a graph illustrating changes in a blood lactic acid/pyruvic acid ratio when an ethanol solution has been administered respectively to a citrate group after administration of a combination drug of potassium citrate and sodium citrate hydrate to SD male rats (8 weeks old) and a control group after administration of purified water to SD male rats (8 weeks old).

### Description of Embodiments

### 1. Food composition

The food composition provided by the present invention may include, as an active ingredient, citric acid or a salt thereof.

Examples of citric acid include citric acid hydrate (e.g., citric acid monohydrate (C₆H₈O7·H₂O)) and anhydrous citric acid.

As the salt of citric acid, an edible or pharmaceutically-acceptable salt for citric acid is preferable, and examples thereof include salts of citric acid with alkali metal, alkaline earth metal, iron, or ammonium ion. More specific examples thereof include monopotassium citrate, dipotassium citrate, tripotassium citrate (in the present specification, tripotassium citrate may simply be referred to as "potassium citrate"), monosodium citrate, disodium citrate, trisodium citrate (in the present specification, trisodium citrate may be simply referred to as "sodium citrate"), calcium citrate, magnesium citrate, ferrous citrate, ferric citrate, sodium ferrous citrate, and ferric ammonium citrate.

As the salt of citric acid, alkali metal salts of citric acid such as monopotassium citrate, dipotassium citrate, potassium citrate, monosodium citrate, disodium citrate, and sodium citrate are preferable. Among them, potassium citrate, sodium citrate, and the like are preferable.

In one embodiment, the salt of citric acid is an edible or pharmaceutically-acceptable salt other than ferrous citrate and ferric citrate.

The salt of citric acid may be a hydrate thereof, or a different salt or a mixture of the hydrate. More specifically, hydrates such as potassium citrate monohydrate (C₆H₅K₃O₇·H₂O) and sodium citrate dihydrate (C₆H₅Na₃O₇·2H₂O) and mixtures thereof can be exemplified. For example, when the alkali metal salt of citric acid as an active ingredient is a mixture of potassium citrate monohydrate (C₆H₅K₃O₇·H₂O) and sodium citrate dihydrate (C₆H₅Na₃O₇·2H₂O), those skilled in the art can appropriately set the mixing ratio of potassium citrate monohydrate (C₆H₅K₃O₇·H₂O) and sodium citrate dihydrate (C₆H₅Na₃O₇·2H₂O). For example, the molar ratio of potassium citrate monohydrate to sodium citrate dihydrate may be in a range of 1 : 0.01 to 1 : 100. The mixing ratio may be about 1 : 1 in molar ratio.

In one embodiment, the mixing ratio of the number of moles of sodium salt of citric acid and the number of moles of potassium salt of citric acid in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

In one embodiment, the mixing ratio of the number of moles of sodium citrate dihydrate (C₆H₅Na₃O₇·2H₂O) and the number of moles of potassium citrate monohydrate (C₆H₅K₃O₇·H₂O) in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

When the active ingredient is a mixture of potassium citrate monohydrate (C₆H₅K₃O₇·H₂O), sodium citrate dihydrate (C₆H₅Na₃O₇·2H₂O), and anhydrous citric acid, the mixing ratio of potassium citrate monohydrate (C₆H₅K₃O₇·H₂O), sodium citrate dihydrate (C₆H₅Na₃O₇·2H₂O), and anhydrous citric acid in the mixture of potassium citrate monohydrate (C₆H₅K₃O₇·H₂O), sodium citrate dihydrate (C₆H₅Na₃O₇·2H₂O), and anhydrous citric acid can be appropriately set by those skilled in the art. For example, as for the molar ratio of potassium citrate monohydrate to sodium citrate dihydrate and anhydrous citric acid, the molar ratio of potassium citrate monohydrate to sodium citrate dihydrate may be in a range of 1 : 0.01 to 1 : 100, and the molar ratio of potassium citrate monohydrate to anhydrous citric acid may be in a range of 1 : 0.01 to 1 : 100. The mixing ratio may be about 2 : 2 : 1 in molar ratio.

Further, other examples of preferred alkali metal salts of citric acid include sodium citrate or hydrates thereof. For example, sodium citrate dihydrate (C₆H₅Na₃O₇·2H₂O) may be used.

Furthermore, other examples of preferred alkali metal salts of citric acid include potassium citrate or hydrates thereof. For example, potassium citrate monohydrate (C₆H₅K₃O₇·H₂O) may be used.

In one embodiment, the mixing ratio of the number of moles of anhydrous citric acid, the number of moles of sodium salt of citric acid, and the number of moles of potassium salt of citric acid in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05 : 1.95-2.05, and particularly preferably 1 : 2 : 2.

In one embodiment, the mixing ratio of the number of moles of anhydrous citric acid, the number of moles of sodium citrate dihydrate (C₆H₅Na₃O₇·2H₂O), and the number of moles of potassium citrate monohydrate (C₆H₅K₃O₇·H₂O) in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05: 1.95-2.05, and particularly preferably 1 : 2 : 2.

In one embodiment, the active ingredient contained in the food composition of the present invention may include a mixture of sodium citrate or a hydrate thereof and potassium citrate or a hydrate thereof.

In another embodiment, the active ingredient contained in the food composition of the present invention may be comprised of only a mixture of sodium citrate or a hydrate thereof and potassium citrate or a hydrate thereof.

In the present specification, when referring to the weight of citric acid and a salt thereof (e.g., potassium citrate monohydrate (C₆H₅K₃O₇·H₂O) and sodium citrate dihydrate (C₆H₅Na₃O₇·2H₂O)), the weight may be dry weight.

An alkali metal salt of citric acid, also referred to as "alkalizing agent", is also known as an agent capable of increasing the HCO₃⁻ level and pH of mammalian (particularly human) body fluids, such as blood or urine.

In the present specification, the term "mammals" include animals classified as Mammalia, and humans are particularly preferable.

In the present specification, the term "alcoholic beverage" means a beverage containing ethanol, and the type thereof is not particularly limited.

In the present specification, the term "alcohol sickness" means an unpleasant physical condition manifested by alcoholic beverage ingestion, which may be accompanied by symptoms such as nausea, vomiting, headache, or dizziness. It is generally understood that alcohol sickness is a symptom caused mainly by acetaldehyde generated in the process of ethanol metabolism after alcoholic beverage ingestion. In the present specification, for convenience, the above-described condition that occurs within 12 hours after the start of alcoholic beverage ingestion is referred to as "alcohol sickness".

In the present specification, the term "hangover" means an unpleasant physical condition manifested by alcoholic beverage ingestion, which may be accompanied by symptoms such as nausea, vomiting, headache, or dizziness. It is generally understood that alcohol sickness is a symptom caused mainly by acetaldehyde generated in the process of ethanol metabolism after alcoholic beverage ingestion. In the present specification, for convenience, the above-described condition that occurs within 24 hours beyond 12 hours after the start of alcoholic beverage ingestion is referred to as "hangover". That is, in the present specification, the terms "alcohol sickness" and "hangover" have no essential difference in their symptoms and causes of onset, but for convenience, they are distinguished by the time when the symptoms occur after the start of alcoholic beverage ingestion.

Note that, in the present specification, the states of "alcohol sickness" and "hangover" and the state of acute alcohol intoxication accompanied by impaired consciousness and the like are clearly distinguished.

The terms "alcohol sickness" and "hangover" can be evaluated by, for example, headache dull,
retching/vomiting, malaise, feeling of drunkenness, stomach discomfort, flushing, headache, chills, or increased pulse rate. Further, the effect of the food composition provided by the present invention on these symptoms can also be evaluated by an increase in the activity value of alcohol dehydrogenase and an increase in the activity value of aldehyde dehydrogenase.

In the present specification, the term "immediately after alcohol ingestion" means within 2 hours, preferably within 1 hour, more preferably within 0.5 hours after the start of alcoholic beverage ingestion, and the like.

In the present specification, the term "improvement" is a concept that includes a process of making "morbid", "abnormal" or "unpleasant" symptoms, conditions or diseases closer to "healthy", "normal" or "comfortable" conditions and methods thereof, and a process of allowing "morbid", "abnormal" or "unpleasant" symptoms, conditions or diseases to return to "healthy", "normal" or "comfortable" conditions or methods thereof. Therefore, in one embodiment, the "improvement" includes that a numerical value as an index of "morbid", "abnormal" or "unpleasant" symptoms or conditions becomes smaller or larger according to the "improvement", and approaches or reaches a normal value.

In the present specification, the term "healthy" indicates a state without an acute or chronic disease or disorder, the term "normal" indicates that a healthy subject is in a normal state, and the term "comfortable" indicates a state without discomfort.

In the present specification, the term "treatment" is a concept that includes the elimination, complete remission, cure or remission of "morbid", "abnormal" or "unpleasant" symptoms, conditions or diseases, and methods thereof, includes suppression of the exacerbation of "morbid", "abnormal" or "unpleasant" symptoms, conditions or diseases, and methods thereof, and also includes the "improvement" described above. Here, the suppression has the meaning described later. In one embodiment, the term "treatment" indicates the elimination, complete remission, cure or remission of "morbid", "abnormal" or "unpleasant" symptoms, conditions or diseases, and methods thereof. In another embodiment, the term "treatment" indicates the elimination, complete remission, cure or remission of "morbid", "abnormal" or "unpleasant" symptoms, conditions or diseases.

In the present specification, the term "prevention" is a concept that includes prevention of the onset of "morbid", "abnormal" or "unpleasant" symptoms, conditions or diseases, and methods thereof.

In the present specification, the term "suppression" is a concept that includes stopping or slowing of the exacerbation or progression of a symptom, condition or disease, and methods thereof, and includes improvement of the symptom, condition or disease, and methods thereof. Here, the improvement has the meaning described above. The above-described term "exacerbation or progression of a symptom, condition or disease" includes the exacerbation or progression of "morbid", "abnormal" or "unpleasant" symptoms, conditions or diseases, and the exacerbation or progression of "morbid", "abnormal" or "unpleasant" symptoms, conditions or diseases from "healthy" "normal " or "comfortable" conditions. In one embodiment, the "suppression" is to stop or slow the exacerbation or progression of a symptom, condition or disease, or methods thereof. In another embodiment, the "suppression" is to stop or slow the exacerbation or progression of a symptom, condition or disease.

Therefore, in the present specification, "suppression of alcohol sickness or hangover due to alcoholic beverage ingestion" can be evaluated by, for example, improvement of headache dull, suppression of retching/vomiting, improvement of malaise, improvement of feeling of drunkenness, improvement of stomach discomfort, improvement of flushing, improvement of headache, improvement of chills, suppression of an increase in pulse rate, and the like, as compared to the pre-ingestion of the food composition of the present invention or the control. Note that, in the present specification, examples of the "control" includes control groups for comparison who have ingested alcoholic beverages without ingesting the food composition of the present invention.

In the present specification, "suppression of an increase in blood alcohol level, suppression of an increase in blood acetaldehyde level, or suppression of an increase in blood acetic acid level due to alcoholic beverage ingestion" can be evaluated by the fact that the blood alcohol level, the blood acetaldehyde level, and the blood acetic acid level each do not increase, are low, tend not to increase, or tend to decrease, as compared to the pre-ingestion of the food composition of the present invention or the control.

The blood alcohol level and the blood acetic acid level can be measured by a known enzyme method. Further, the blood acetaldehyde level can be measured by a high performance liquid chromatography (HPLC) method.

In the present specification, "suppression of a decrease in blood pyruvic acid level due to alcoholic beverage ingestion" can be evaluated by the fact that the blood pyruvic acid level does not decrease, is high, tends not to decrease, or tends to increase, as compared to pre-ingestion of the food composition of the present invention or control.

The blood pyruvic acid level can be measured by a known enzyme method.

In the present specification, "suppression of abnormal blood lactic acid/pyruvic acid ratio due to alcoholic beverage ingestion" can be evaluated by the fact that the blood lactic acid/pyruvic acid ratio due to alcoholic beverage ingestion is in a range of -20% to +20%, preferably in a range of -15% to +15%, more preferably in a range of -10% to +10%, and still more preferably in a range of -5% to +5%, as compared to the blood lactic acid/pyruvic acid ratio before alcohol ingestion, or can be evaluated by that fact that the blood lactic acid/pyruvic acid ratio due to alcoholic beverage ingestion tends to deviate from or approach to the blood lactic acid/pyruvic acid ratio in the control, as compared to pre-ingestion of the food composition of the present invention.

Since the blood lactic acid level can be measured by a known enzyme method, the ratio of the blood lactic acid level to the blood pyruvic acid level measured by the above method can be calculated.

In the present specification, "suppression of an increase in blood alcohol level immediately after alcohol ingestion" can be evaluated by the fact that the blood alcohol level does not increase within 2 hours, preferably within 1 hour, more preferably within 0.5 hour after the start of alcoholic beverage ingestion or the like, as compared to the pre-ingestion of the food composition of the present invention or the control, or the rate of increase in concentration per unit time is small.

In the present specification, "suppression of alcohol absorption" can be evaluated by the fact that the blood alcohol level does not increase, is low, tends not to increase, or tends to decrease, as compared to pre-ingestion of the food composition of the present invention or control.

In the present specification, "promotion of alcohol metabolism" can be evaluated by the fact that the blood alcohol level does not increase, is low, tends not to increase, or tends to decrease, as compared to pre-ingestion of the food composition of the present invention or control.

In one embodiment, the ingestion of the food composition provided by the present invention exerts an effect such as suppression of an increase in blood alcohol level, suppression of an increase in blood acetaldehyde level, or suppression of an increase in blood acetic acid level due to alcoholic beverage ingestion, compared to the pre-ingestion or the control, and thus the food composition provided by the present invention is useful in suppressing alcohol sickness or hangover due to alcoholic beverage ingestion.

One aspect of suppression of the increase in blood alcohol level, suppression of the increase in blood acetaldehyde level, or suppression of the increase in blood acetic acid level due to alcoholic beverage ingestion is suppression of absorption of alcohol in the ingested alcoholic beverage, and another aspect is suppression of transfer of alcohol in the ingested alcoholic beverage into the blood.

Another aspect of suppression of the increase in blood alcohol level, suppression of the increase in blood acetaldehyde level, or suppression of the increase in blood acetic acid level due to alcoholic beverage ingestion is a promotion of metabolism of alcohol absorbed due to alcoholic beverage ingestion.

In one embodiment, the ingestion of the food composition provided by the present invention exerts an effect such as suppression of a decrease in blood pyruvic acid level due to alcoholic beverage ingestion, compared to the pre-ingestion or the control, and thus the food composition provided by the present invention is useful in suppressing alcohol sickness or hangover due to alcoholic beverage ingestion.

In one embodiment, the ingestion of the food composition provided by the present invention exerts an effect such as suppression of an abnormal blood lactic acid/pyruvic acid ratio due to alcoholic beverage ingestion, compared to the pre-ingestion or the control, and thus the food composition provided by the present invention is useful in suppressing alcohol sickness or hangover due to alcoholic beverage ingestion.

In one embodiment, the ingestion of the food composition provided by the present invention exerts an effect such as suppression of an increase in blood alcohol level immediately after alcohol ingestion, compared to the pre-ingestion or the control, and thus the food composition provided by the present invention is useful in suppressing alcohol sickness or hangover due to alcoholic beverage ingestion.

In one embodiment, the ingestion of the food composition provided by the present invention exerts an effect such as suppression of alcohol absorption, compared to the pre-ingestion or the control, and thus the food composition provided by the present invention is useful in suppressing alcohol sickness or hangover due to alcoholic beverage ingestion.

In one embodiment, the ingestion of the food composition provided by the present invention exerts an effect such as suppression of alcohol metabolism, compared to the pre-ingestion or the control, and thus the food composition provided by the present invention is useful in suppressing alcohol sickness or hangover due to alcoholic beverage ingestion.

In one embodiment, the ingestion of the food composition provided by the present invention exerts an effect, such as increased blood ornithine level, increased blood taurine level, increased blood cystine level, or increased blood nicotine amide level, as compared to the pre-ingestion or the control. Accordingly, the food composition provided by the present invention is useful in suppressing alcohol sickness or hangover due to alcoholic beverage ingestion.

In one embodiment, the ingestion of the food composition provided by the present invention suppresses the alcohol absorption and promotes the alcohol metabolism.

The content of citric acid or a salt thereof in the food composition provided by the present invention can be appropriately determined depending on the type of food. Examples of food compositions include foods for specified health use, foods with functional claims, foods for hospital patients, and supplements. The form of these food compositions is not particularly limited as long as it includes citric acid or a salt thereof in an effective amount that exerts the above effect and is orally ingestible. The food compositions may be in the form of a normal food or drink, or may be formulated and provided as a formulation suitable for oral administration, such as a tablet, a capsule, or a liquid formulation. In the present specification, with respect to the constitution and production method of these formulations, the formulation technology known per se in the field of pharmaceutical formulation technology (hereinafter, may be referred to as "pharmaceutical field") can be applied to the formulation of the food composition provided by the present invention.

The formulation of the food composition provided by the present invention may be prepared by mixing citric acid or a salt thereof as it is, or may be prepared by mixing citric acid or a salt thereof with an edible carrier, such as an excipient (e.g., lactose, D-mannitol, crystalline cellulose, or glucose), a binder (e.g., hydroxypropylcellulose (HPC), gelatin or polyvinylpyrrolidone (PVP)), a lubricant (e.g., magnesium stearate or talc), a disintegrant (e.g., starch or carboxymethylcellulose calcium (CMC-Ca)), a diluent (e.g., water or physiological saline), and, if necessary, other additives (e.g., a pH adjuster, a surfactant, a solubilizing agent, a preservative, an emulsifier, an isotonic agent, or a stabilizer). The formulation of the food composition provided by the present invention can be produced by applying a method known in the pharmaceutical or food fields. The dosage form of the formulation of the food composition provided by the present invention may be a formulation that can be orally ingested, such as tablets, capsules, and liquid formulations. For example, in order to make tablets, citric acid or a salt thereof may be mixed with an excipient (e.g., lactose, D-mannitol, crystalline cellulose or glucose), a disintegrant (e.g., starch or carboxymethylcellulose calcium (CMC)-Ca)), a binder (e.g., hydroxypropylcellulose (HPC), gelatin, or polyvinylpyrrolidone (PVP)), a lubricant (e.g., magnesium stearate or talc), and the like for formulation. In the present specification, when the active ingredient of the food composition is citric acid or a salt thereof, these ingredients are not included in the carrier.

The amount of the active ingredient in the food composition provided by the present invention can be appropriately set. The content of citric acid or a salt thereof in the food composition provided by the present invention may be in a range of 10 to 95% by weight, preferably in a range of 30 to 90% by weight, and more preferably in a range of 60 to 85% by weight relative to the food composition.

The ingestion dose of citric acid or a salt thereof as an active ingredient is appropriately determined depending on the type of the active ingredient, the method of ingestion, the age, weight, sex, and symptoms of the subject for ingestion, susceptibility to the active ingredient, and the like. The timing and dose of ingestion may be adjusted according to the type of symptom and the state of improvement or suppression thereof.

The daily ingestion dose of citric acid or a salt thereof may be, for example, in a range of 0.1 to 10 g, 1 to 5 g, or 1 to 3 g. In one embodiment, the formulation of the food composition is an active ingredient, its content or dosage form that is distinguishable from a pharmaceutical formulation approved as a pharmaceutical product for improving acidic urine in gout or hyperuricemia.

In one embodiment, for example, in the case of foods for specified health use, nutritional supplements, foods with functional claims or foods for hospital patients, potassium citrate monohydrate and sodium citrate dihydrate may be contained in a total amount of 1/3 of 1 to 3 g per serving. When foods for specified health use, nutritional supplements, foods with functional claims, foods for hospital patients or supplements are provided as tablets, for example, 70 to 80% by weight of citric acid or a salt thereof may be contained in a tablet (300 mg to 600 mg).

In one embodiment, when the active ingredient of the food composition (e.g., tablet) provided by the present invention is an alkali metal salt of citric acid (e.g., potassium citrate or a hydrate thereof (e.g., potassium citrate monohydrate); sodium citrate or a hydrate thereof (e.g., sodium citrate dihydrate); a mixture of potassium citrate or a hydrate thereof and sodium citrate or a hydrate thereof; or a mixture of potassium citrate monohydrate and sodium citrate dihydrate, the food composition provided by the present invention (e.g., tablet) may contain anhydrous citric acid as a stabilizer.

When the food composition provided by the present invention is not formulated and is provided in the form of a normal food or drink, it can be appropriately produced by those skilled in the art depending on the type of the food, and can be produced, for example, by blending a food material with citric acid or a salt thereof (e.g., potassium citrate and/or sodium citrate).

Examples of forms of the food or drink include liquid or milky or pasty foods such as beverage, soy sauce, milk, yogurt, and fermented soybean paste; semi-solid foods such as jelly and gummy candy; solid foods such as candy, gum, soybean curd, and supplement; and powdered foods.

Examples of beverages include fruit juice and fruit beverages, coffee beverages, oolong tea beverages, green tea beverages, black tea beverages, barley tea beverages, vegetable beverages, soft drinks such as carbonated beverages, fruit extract-containing beverages, vegetable extract-containing juices, flavored water, sports drinks, and diet drinks.

To beverages, additives such as antioxidants, fragrances, various esters, organic acids, organic acid salts, inorganic acids, inorganic acid salts, inorganic salts, pigments, emulsifiers, preservatives, seasoning agents, sweeteners, acidulants, fruit juice extracts, vegetable extracts, flower honey extracts, pH adjusters, and quality stabilizers can be added singly or in combination.

In one embodiment, the food composition provided by the present invention can be applied to a subject in need of suppression of alcohol sickness or hangover due to alcoholic beverage ingestion.

In one embodiment, the subject in need of suppression of alcohol sickness or hangover due to alcoholic beverage ingestion means a mammal (particularly a human) who does not have "morbid", "abnormal" or "unpleasant" symptoms, conditions or diseases, such as alcohol sickness or hangover due to alcoholic beverage ingestion; or a subject who has "morbid", "abnormal" or "unpleasant" symptoms, such as alcohol sickness or hangover, but does not seem to have any disease or disorder, i.e., a subject in a "healthy" condition. The food composition provided by the present invention can be applied to maintain or enhance "healthy", "normal" or "comfortable" conditions, or make the "comfortable" condition more comfortable, or improve the "morbid", "abnormal" or "unpleasant" symptoms, such as alcohol sickness or hangover.

In one embodiment, the food composition provided by the present invention can be applied to "an individual who is scheduled to ingest an alcoholic beverage (healthy individual)", "an individual who may ingest an alcoholic beverage (healthy individual)", "an individual who ingested an alcoholic beverage (healthy individual)", "an individual who ingested an excessive amount of an alcoholic beverage (healthy individual)" or "an individual who has symptoms of alcohol sickness or hangover due to alcoholic beverage ingestion (healthy individual)", in order to suppress alcohol sickness or hangover due to alcoholic beverage ingestion. In this case, the above ingestion dose of the food composition provided by the present invention may be ingested in advance before alcoholic beverage ingestion. For example, the ingestion dose can be ingested, 3 hours before, more preferably 0.5 hour to 2 hours before the scheduled time of alcoholic beverage ingestion, or after alcoholic beverage ingestion and before onset of symptoms of alcohol sickness or hangover, or when alcoholic beverage ingestion causes symptoms of alcohol sickness or hangover.

In one embodiment, an effective amount of the food composition provided by the present invention is administered to a subject in need of suppression of an increase in blood alcohol level, an increase in blood acetaldehyde level, or an increase in blood acetic acid level due to alcoholic beverage ingestion.

In one embodiment, an effective amount of the food composition provided by the present invention is administered to a subject in need of suppression of a decrease in blood pyruvic acid level due to alcoholic beverage ingestion.

In one embodiment, an effective amount of the food composition provided by the present invention is administered to a subject in need of suppression of an abnormal blood lactic acid/pyruvic acid ratio due to alcoholic beverage ingestion.

In one embodiment, an effective amount of the food composition provided by the present invention is administered to a subject in need of suppression of an increase in blood alcohol level immediately after alcohol ingestion.

In one embodiment, an effective amount of the food composition provided by the present invention is administered to a subject in need of suppression of alcohol absorption.

In one embodiment, an effective amount of the food composition provided by the present invention is administered to a subject in need of promotion of alcohol metabolism.

In one embodiment, the effective amount of the food composition provided by the present invention is administered to a subject in need of an increase in blood ornithine level, blood serotonin level, blood taurine level, blood cystine level, or blood nicotine amide level.

In one embodiment, a subject in need of suppression of an increase in blood alcohol level, an increase in blood acetaldehyde level, or an increase in blood acetic acid level due to alcoholic beverage ingestion; a subject in need of suppression of a decrease in blood pyruvic acid level due to alcoholic beverage ingestion; a subject in need of suppression of an abnormal blood lactic acid/pyruvic acid ratio due to alcoholic beverage ingestion; a subject in need of suppression of an increase in blood alcohol level immediately after alcohol ingestion; a subject in need of suppression of alcohol absorption; a subject in need of suppression of alcohol metabolism; and a subject in need of increased blood ornithine level, increased blood taurine level, increased blood cystine level, or increased blood nicotine amide level may be the same as the subject in need of suppression of alcohol sickness or hangover due to alcoholic beverage ingestion.

A food composition, that is applied to a subject who does not have "morbid", "abnormal" or "unpleasant" symptoms, conditions or diseases; or a subject who has "morbid", "abnormal" or "unpleasant" symptoms, but does not seem to have any disease or disorder, i.e., a subject in a "healthy" condition in order to maintain or enhance "healthy", "normal" or "comfortable" conditions, or make the "comfortable" condition more comfortable, or improve the "morbid", "abnormal" or "unpleasant" symptoms, may be particularly referred to as "food with functional claims".

Therefore, embodiments of the food composition according to the present invention include the following:
<1-1> A food composition for suppressing alcohol sickness or hangover due to alcoholic beverage ingestion in mammals (particularly humans), including, as an active ingredient, citric acid or a salt thereof;
<1-2> A method for suppressing alcohol sickness or hangover due to alcoholic beverage ingestion, including allowing a subject in need of suppression of alcohol sickness or hangover due to alcoholic beverage ingestion to ingest a food composition containing an effective amount of citric acid or a salt thereof; and
<1-3> Use of citric acid or a salt thereof for producing a food composition for suppressing alcohol sickness or hangover due to alcoholic beverage ingestion.

Another aspect of the embodiments of the food composition according to the present invention includes the following:
<2-1> A food composition for suppressing an increase in blood alcohol level, an increase in blood acetaldehyde level, or an increase in blood acetic acid level due to alcoholic beverage ingestion in mammals (particularly humans), including, as an active ingredient, citric acid or a salt thereof;
<2-2> A method for suppressing an increase in blood alcohol level, an increase in blood acetaldehyde level, or an increase in blood acetic acid level due to alcoholic beverage ingestion, including allowing a subject in need of suppression of an increase in blood alcohol level, an increase in blood acetaldehyde level, or an increase in blood acetic acid level due to alcoholic beverage ingestion to ingest a food composition containing an effective amount of citric acid or a salt thereof; and
<2-3> Use of citric acid or a salt thereof for producing a food composition for suppressing an increase in blood alcohol level, an increase in blood acetaldehyde level, or an increase in blood acetic acid level due to alcoholic beverage ingestion.

Another aspect of the embodiments of the food composition according to the present invention includes the following:
<3-1> A food composition for suppressing a decrease in blood pyruvic acid level due to alcoholic beverage ingestion in mammals (particularly humans), including, as an active ingredient, citric acid or a salt thereof;
<3-2> A method for suppressing a decrease in blood pyruvic acid level due to alcoholic beverage ingestion, including allowing a subject in need of suppression of a decrease in blood pyruvate level due to alcoholic beverage ingestion to ingest a food composition containing an effective amount of citric acid or a salt thereof; and
<3-3> Use of citric acid or a salt thereof for producing a food composition for suppressing a decrease in blood pyruvic acid level due to alcoholic beverage ingestion.

Another aspect of the embodiments of the food composition according to the present invention includes the following:
<4-1> A food composition for suppressing an abnormal blood lactic acid/pyruvic acid ratio due to alcoholic beverage ingestion in mammals (particularly humans), including, as an active ingredient, citric acid or a salt thereof;
<4-2> A method for suppressing an abnormal blood lactic acid/pyruvic acid ratio due to alcoholic beverage ingestion, including allowing a subject in need of suppression of abnormal blood lactic acid/pyruvic acid ratio due to alcoholic beverage ingestion to ingest a food composition containing an effective amount of citric acid or a salt thereof; and
<4-3> Use of citric acid or a salt thereof for producing a food composition for suppressing an abnormal blood lactic acid/pyruvic acid ratio due to alcoholic beverage ingestion.

Another aspect of the embodiments of the food composition according to the present invention includes the following:
<5-1> A food composition for suppressing an increase in blood alcohol level immediately after alcohol ingestion in mammals (particularly humans), including, as an active ingredient, citric acid or a salt thereof;
<5-2> A method for suppressing an increase in blood alcohol level immediately after alcohol ingestion, including allowing a subject in need of suppression of an increase in blood alcohol level immediately after alcohol ingestion to ingest a food composition containing an effective amount of citric acid or a salt thereof; and
<5-3> Use of citric acid or a salt thereof for producing a food composition for suppressing an increase in blood alcohol level immediately after alcohol ingestion.

Another aspect of the embodiments of the food composition according to the present invention includes the following:
<6-1> A food composition for suppressing alcohol absorption in mammals (particularly humans), including, as an active ingredient, citric acid or a salt thereof;
<6-2> A method for suppressing alcohol absorption, including allowing a subject in need of suppression of alcohol absorption to ingest a food composition containing an effective amount of citric acid or a salt thereof; and <6-3> Use of citric acid or a salt thereof for producing a food composition for suppressing alcohol absorption.

Another aspect of the embodiments of the food composition according to the present invention includes the following:
<7-1> A food composition for promoting alcohol metabolism in mammals (particularly humans), including, as an active ingredient, citric acid or a salt thereof;
<7-2> A method for promoting alcohol metabolism, including allowing a subject in need of promotion of alcohol metabolism to ingest a food composition containing an effective amount of citric acid or a salt thereof; and
<7-3> Use of citric acid or a salt thereof for producing a food composition for promoting alcohol metabolism.

Another aspect of the embodiments of the food composition according to the present invention includes the following:
<8-1> A food composition for increasing a blood ornithine level, a blood serotonin level, a blood taurine level, a blood cystine level, or a blood nicotine amide level in mammals (particularly humans), including, as an active ingredient, citric acid or a salt thereof;
<8-2> A method for increasing a blood ornithine level, a blood serotonin level, a blood taurine level, a blood cystine level, or a blood nicotine amide level, including allowing a subject in need of an increase in blood ornithine level, blood serotonin level, blood taurine level, blood cystine level, or blood nicotine amide level to ingest an effective amount of a food composition containing citric acid or a salt thereof; and
<8-3> Use of citric acid or a salt thereof for producing a food composition for increasing a blood ornithine level, a blood serotonin level, a blood taurine level, a blood cystine level, or a blood nicotine amide level.

The blood ornithine level, blood serotonin level, blood taurine level, blood cystine level, and blood nicotine amide level can be measured by a known HPLC method, enzyme method, enzyme immunoassay method, or the like.

Preferably, the food composition according to the present invention is a food composition whose package, container, or instruction indicates an effect of suppressing alcohol sickness or hangover due to alcoholic beverage ingestion; an effect of suppressing an increase in blood alcohol level, an increase in blood acetaldehyde level, or an increase in blood acetic acid level due to alcoholic beverage ingestion; an effect of suppressing a decrease in blood pyruvic acid level due to alcoholic beverage ingestion; an effect of suppressing an abnormal blood lactic acid/pyruvic acid ratio due to alcoholic beverage ingestion; an effect of suppressing an increase in blood alcohol level immediately after alcohol ingestion; an effect of suppressing alcohol absorption; an effect of promoting alcohol metabolism; or an effect of increasing a blood ornithine level, a blood serotonin level, a blood taurine level, a blood cystine level, or a blood nicotine amide level.

### 2. Pharmaceutical composition

The pharmaceutical composition provided by the present invention may include, as an active ingredient, citric acid or a salt thereof. Citric acid or a salt thereof included in the pharmaceutical composition is synonymous with citric acid or a salt thereof described as an active ingredient of the food composition, and the specific examples thereof are the same as those described above.

In one embodiment, when the active ingredient of the pharmaceutical composition (e.g., tablet) provided by the present invention is an alkali metal salt of citric acid (e.g., potassium citrate or a hydrate thereof (e.g., potassium citrate monohydrate); sodium citrate or a hydrate thereof (e.g., sodium citrate dihydrate); a mixture of potassium citrate or a hydrate thereof and sodium citrate or a hydrate thereof; or a mixture of potassium citrate monohydrate and sodium citrate dihydrate, the pharmaceutical composition provided by the present invention (e.g., tablet) may contain anhydrous citric acid as a stabilizer.

In one embodiment, the administration of the pharmaceutical composition provided by the present invention leads to suppression of alcohol sickness or hangover due to alcoholic beverage ingestion in mammals (particularly humans), compared to pre-administration or control.

In one embodiment, the administration of the pharmaceutical composition provided by the present invention leads to suppression of an increase in blood alcohol level, an increase in blood acetaldehyde level, or an increase in blood acetic acid level due to alcoholic beverage ingestion in mammals (particularly humans), compared to the pre-administration or the control.

One aspect of suppression of the increase in blood alcohol level, suppression of the increase in blood acetaldehyde level, or suppression of the increase in blood acetic acid level due to alcoholic beverage ingestion is suppression of absorption of alcohol in the ingested alcoholic beverage, and another aspect is suppression of transfer of alcohol in the ingested alcoholic beverage into the blood.

Another aspect of suppression of the increase in blood alcohol level, suppression of the increase in blood acetaldehyde level, or suppression of the increase in blood acetic acid level due to alcoholic beverage ingestion is a promotion of metabolism of alcohol absorbed due to alcoholic beverage ingestion.

In one embodiment, the administration of the pharmaceutical composition provided by the present invention exerts an effect such as suppression of a decrease in blood pyruvic acid level due to alcoholic beverage ingestion, compared to the pre-administration or the control, and thus the pharmaceutical composition provided by the present invention is useful in suppressing alcohol sickness or hangover due to alcoholic beverage ingestion.

In one embodiment, the administration of the pharmaceutical composition provided by the present invention exerts an effect such as suppression of an abnormal blood lactic acid/pyruvic acid ratio due to alcoholic beverage ingestion, compared to the pre-administration or the control, and thus the pharmaceutical composition provided by the present invention is useful in suppressing alcohol sickness or hangover due to alcoholic beverage ingestion.

In one embodiment, the administration of the pharmaceutical composition provided by the present invention exerts an effect such as suppression of an increase in blood alcohol level immediately after alcohol ingestion, compared to the pre-administration or the control, and thus the pharmaceutical composition provided by the present invention is useful in suppressing alcohol sickness or hangover due to alcoholic beverage ingestion.

In one embodiment, the administration of the pharmaceutical composition provided by the present invention exerts an effect such as suppression of alcohol absorption, compared to the pre-administration or the control, and thus the pharmaceutical composition provided by the present invention is useful in suppressing alcohol sickness or hangover due to alcoholic beverage ingestion.

In one embodiment, the administration of the pharmaceutical composition provided by the present invention exerts an effect such as suppression of alcohol metabolism, compared to the pre-administration or the control, and thus the pharmaceutical composition provided by the present invention is useful in suppressing alcohol sickness or hangover due to alcoholic beverage ingestion.

In one embodiment, the administration of the pharmaceutical composition provided by the present invention exerts an effect, such as increased blood ornithine level, increased blood taurine level, increased blood cystine level, or increased blood nicotine amide level, as compared to the pre-administration or the control. Accordingly, the pharmaceutical composition provided by the present invention is useful in suppressing alcohol sickness or hangover due to alcoholic beverage ingestion.

The pharmaceutical composition provided by the present invention is orally or parenterally administered to a human or another mammal, and examples of parenteral administration include intravenous administration, subcutaneous administration, intramuscular administration, intraarticular administration, and transmucosal administration, transdermal administration, nasal administration, rectal administration, intrathecal administration, intraperitoneal administration, and local administration.

The pharmaceutical composition provided by the present invention may be prepared by using citric acid or a salt thereof as it is, or by mixing citric acid or a salt thereof with a pharmaceutically acceptable carrier such as an excipient, a binder, a lubricant, a disintegrant, a diluent, and if necessary, other additives, and may a formulation such as a tablet, a capsule, a suspension, an injection, or a suppository.

The excipient, binder, lubricant, disintegrant, diluent, and other additives described in the food composition can be used for the excipient, binder, lubricant, disintegrant, diluent, and other additives as described above. Further, the food composition provided by the present invention can be formulated into a tablet, a capsule, a suppository, or the like suitable for oral ingestion, whereas the pharmaceutical composition provided by the present invention can be formulated into a dosage form suitable for parenteral administration such as an injection or a suppository, in addition to a dosage form suitable for oral ingestion like the food composition.

In one embodiment, the pharmaceutical composition provided by the present invention is a tablet. The content of citric acid or a salt thereof in the tablet of the pharmaceutical composition provided by the present invention may be in a range of 10 to 95% by weight, preferably in a range of 30 to 90% by weight, and more preferably in a range of 60 to 85% by weight relative to the tablet.

The administered dose of citric acid or a salt thereof as an active ingredient is appropriately determined depending on the type of the active ingredient, the administration method, the age, weight, sex, and symptoms of the subject to be administered, the susceptibility to the active ingredient, and the like. The timing and dose of administration may be adjusted according to the type of symptom and the state of improvement or suppression thereof.

The daily administered dose of citric acid or a salt thereof may be, for example, in a range of 0.1 to 10 g, 1 to 5 g, or 1 to 3 g.

In one embodiment, in the pharmaceutical composition provided by the present invention, as the alkali metal salt of citric acid, each of potassium citrate monohydrate and sodium citrate dihydrate is orally administered in a dose of 0.5 to 1.5 g/day for a total of 1 to 3 g/day, and is administered daily in 1 to 5 divided doses, preferably 3 divided doses.

In one embodiment, one dosing unit (preferably one tablet) of the pharmaceutical composition provided by the present invention contains, as alkali metal salts of citric acid, 231.5 mg of potassium citrate monohydrate and 195.0 mg of sodium citrate dihydrate, and three to six dosage units are orally administered daily in 3 divided doses.

In one embodiment, the pharmaceutical composition provided by the present invention is administered before alcohol ingestion (e.g., immediately before alcohol ingestion, from 0 to 30 minutes before alcohol ingestion, from 0 to 1 hour before alcohol ingestion, from 0 to 3 hours before alcohol ingestion).

The term "ingestion" described in "1. Food composition" above can also be applied to the "pharmaceutical composition" according to the present invention. Further, regarding the "pharmaceutical composition" according to the present invention, the term "ingestion" may be replaced with "administration". Therefore, for example, the term "ingest", "allowing a subject to ingest", "ingested", or the like can be changed and replaced with "administer", "administered", or the like, depending on the context. The technology applied to the formulated food composition can also be applied to the pharmaceutical composition.

In one embodiment, an effective amount of the pharmaceutical composition provided by the present invention is administered to a subject (e.g., a human or another mammal) who needs to suppress alcohol sickness or hangover due to alcoholic beverage ingestion.

In one embodiment, an effective amount of the pharmaceutical composition provided by the present invention is administered to a subject in need of treatment or prevention of alcohol sickness or hangover due to alcoholic beverage ingestion, for example, a subject in need of suppression of at least one symptom selected from the group consisting of headache dull, retching/vomiting, malaise, feeling of drunkenness, stomach discomfort, abdominal pain, gastrointestinal disorders, flushing, headache, chills, and increased pulse rate.

In one embodiment, an effective amount of the pharmaceutical composition provided by the present invention is administered to a subject in need of suppression of an increase in blood alcohol level, an increase in blood acetaldehyde level, or an increase in blood acetic acid level due to alcoholic beverage ingestion.

In one embodiment, an effective amount of the pharmaceutical composition provided by the present invention is administered to a subject in need of suppression of a decrease in blood pyruvic acid level due to alcoholic beverage ingestion.

In one embodiment, an effective amount of the pharmaceutical composition provided by the present invention is administered to a subject in need of suppression of abnormal blood lactic acid/pyruvic acid ratio due to alcoholic beverage ingestion.

In one embodiment, an effective amount of the pharmaceutical composition provided by the present invention is administered to a subject in need of suppression of an increase in blood alcohol level immediately after alcohol ingestion.

In one embodiment, an effective amount of the pharmaceutical composition provided by the present invention is administered to a subject in need of suppression of alcohol absorption.

In one embodiment, an effective amount of the pharmaceutical composition provided by the present invention is administered to a subject in need of promotion of alcohol metabolism.

In one embodiment, the effective amount of the pharmaceutical composition provided by the present invention is administered to a subject in need of an increase in blood ornithine level, blood serotonin level, blood taurine level, blood cystine level, or blood nicotine amide level.

In one embodiment, a subject in need of suppression of an increase in blood alcohol level, an increase in blood acetaldehyde level, or an increase in blood acetic acid level due to alcoholic beverage ingestion; a subject in need of suppression of a decrease in blood pyruvic acid level due to alcoholic beverage ingestion; a subject in need of suppression of an abnormal blood lactic acid/pyruvic acid ratio due to alcoholic beverage ingestion; a subject in need of suppression of an increase in blood alcohol level immediately after alcohol ingestion; a subject in need of suppression of alcohol absorption; a subject in need of suppression of alcohol metabolism; and a subject in need of increased blood ornithine level, increased blood taurine level, increased blood cystine level, or increased blood nicotine amide level may be the same as the subject in need of suppression of alcohol sickness or hangover due to alcoholic beverage ingestion.

Examples of other embodiments of the present invention include following:
(1-1) A method for suppressing alcohol sickness or hangover due to alcoholic beverage ingestion, including administering a pharmaceutical composition containing an effective amount of citric acid or a salt thereof, to a subject in need of suppression of alcohol sickness or hangover due to alcoholic beverage ingestion;
(1-2) A method for suppressing an increase in blood alcohol level, an increase in blood acetaldehyde level, or an increase in blood acetic acid level due to alcoholic beverage ingestion, including administering a pharmaceutical composition containing an effective amount of citric acid or a salt thereof, to a subject in need of suppression of an increase in blood alcohol level, an increase in blood acetaldehyde level, or an increase in blood acetic acid level due to alcoholic beverage ingestion;
(1-3) A method for suppressing a decrease in blood pyruvic acid level due to alcoholic beverage ingestion, including administering a pharmaceutical composition containing an effective amount of citric acid or a salt thereof, to a subject in need of suppression of a decrease in blood pyruvic acid level due to alcoholic beverage ingestion;
(1-4) A method for suppressing an abnormal blood lactic acid/pyruvic acid ratio due to alcoholic beverage ingestion, including administering a pharmaceutical composition containing an effective amount of citric acid or a salt thereof, to a subject in need of suppression of abnormal blood lactic acid/pyruvic acid ratio due to alcoholic beverage ingestion;
(1-5) A method for suppressing an increase in blood alcohol level immediately after alcohol ingestion, including administering a pharmaceutical composition containing an effective amount of citric acid or a salt thereof, to a subject in need of suppression of an increase in blood alcohol level immediately after alcohol ingestion;
(1-6) A method for suppressing alcohol absorption, including administering a pharmaceutical composition containing an effective amount of citric acid or a salt thereof, to a subject in need of suppression of alcohol absorption;
(1-7) A method for promoting alcohol metabolism, including administering an effective amount of a pharmaceutical composition containing citric acid or a salt thereof to a subject in need of promotion of alcohol metabolism;
(1-8) A method for increasing a blood ornithine level, a blood serotonin level, a blood taurine level, a blood cystine level, and a blood nicotine amide level, including administering an effective amount of a pharmaceutical composition containing citric acid or a salt thereof, to a subject in need of an increase in blood ornithine level, blood serotonin level, blood taurine level, blood cystine level, or blood nicotine amide level.
(1-9) The method according to any one of (1-1) to (1-8), wherein the subject is a patient suffering from kidney disease;
(2-1) A pharmaceutical composition including citric acid or a salt thereof for use in suppressing alcohol sickness or hangover due to alcoholic beverage ingestion;
(2-2) A pharmaceutical composition including citric acid or a salt thereof for use in suppressing alcohol sickness or hangover due to alcoholic beverage ingestion in a patient with kidney disease;
(2-3) A pharmaceutical composition including citric acid or a salt thereof for suppressing an increase in blood alcohol level, an increase in blood acetaldehyde level, or an increase in blood acetic acid level due to alcoholic beverage ingestion;
(2-4) A pharmaceutical composition including citric acid or a salt thereof for suppressing an increase in blood alcohol level, an increase in blood acetaldehyde level, or an increase in blood acetic acid level due to alcoholic beverage ingestion in a patient with kidney disease;
(2-5) A pharmaceutical composition including citric acid or a salt thereof for use in suppressing a decrease in blood pyruvic acid level due to alcoholic beverage ingestion;
(2-6) A pharmaceutical composition including citric acid or a salt thereof for use in suppressing a decrease in blood pyruvic acid level due to alcoholic beverage ingestion in a patient with kidney disease;
(2-7) A pharmaceutical composition including citric acid or a salt thereof for use in suppressing an abnormal blood lactic acid/pyruvic acid ratio due to alcoholic beverage ingestion;
(2-8) A pharmaceutical composition including citric acid or a salt thereof for use in suppressing an abnormal blood lactic acid/pyruvic acid ratio due to alcoholic beverage ingestion in a patient with kidney disease;
(2-9) A pharmaceutical composition including citric acid or a salt thereof for use in suppressing an increase in blood alcohol level immediately after alcohol ingestion;
(2-10) A pharmaceutical composition including citric acid or a salt thereof for use in suppressing an increase in blood alcohol level immediately after alcohol ingestion in a patient with kidney disease;
(2-11) A pharmaceutical composition including citric acid or a salt thereof for use in suppressing alcohol absorption;
(2-12) A pharmaceutical composition including citric acid or a salt thereof for use in suppressing alcohol absorption in a patient with kidney disease;
(2-13) A pharmaceutical composition including citric acid or a salt thereof for use in promoting alcohol metabolism;
(2-14) A pharmaceutical composition including citric acid or a salt thereof for use in promoting alcohol metabolism in a patient with kidney disease;
(2-15) A pharmaceutical composition including citric acid or a salt thereof for use in increasing a blood ornithine level, a blood serotonin level, a blood taurine level, a blood cystine level, or a blood nicotine amide level;
(2-16) A pharmaceutical composition including citric acid or a salt thereof for use in increasing a blood ornithine level, a blood serotonin level, a blood taurine level, a blood cystine level, or a blood nicotine amide level in a patient with kidney disease;
(3-1) Use of citric acid or a salt thereof for producing a pharmaceutical composition for suppressing alcohol sickness or hangover due to alcoholic beverage ingestion;
(3-2) Use of citric acid or a salt thereof for producing a pharmaceutical composition for suppressing alcohol sickness or hangover due to alcoholic beverage ingestion in a patient with kidney disease;
(3-3) Use of citric acid or a salt thereof for producing a pharmaceutical composition for suppressing an increase in blood alcohol level, an increase in blood acetaldehyde level, or an increase in blood acetic acid level due to alcoholic beverage ingestion;
(3-4) Use of citric acid or a salt thereof for producing a pharmaceutical composition for suppressing an increase in blood alcohol level, an increase in blood acetaldehyde level, or an increase in blood acetic acid level due to alcoholic beverage ingestion in a patient with kidney disease;
(3-5) Use of citric acid or a salt thereof for producing a pharmaceutical composition for suppressing a decrease in blood pyruvic acid level due to alcoholic beverage ingestion;
(3-6) Use of citric acid or a salt thereof for producing a pharmaceutical composition for suppressing a decrease in blood pyruvic acid level due to alcoholic beverage ingestion in a patient with kidney disease;
(3-7) Use of citric acid or a salt thereof for producing a pharmaceutical composition for suppressing an abnormal blood lactic acid/pyruvic acid ratio due to alcoholic beverage ingestion;
(3-8) Use of citric acid or a salt thereof for producing a pharmaceutical composition for suppressing an abnormal blood lactic acid/pyruvic acid ratio due to alcoholic beverage ingestion in a patient with kidney disease;
(3-9) Use of citric acid or a salt thereof for producing a pharmaceutical composition for suppressing an increase in blood alcohol level immediately after alcohol ingestion;
(3-10) Use of citric acid or a salt thereof for producing a pharmaceutical composition for suppressing an increase in blood alcohol level immediately after alcohol ingestion in a patient with kidney disease;
(3-11) Use of citric acid or a salt thereof for producing a pharmaceutical composition for suppressing alcohol absorption;
(3-12) Use of citric acid or a salt thereof for producing a pharmaceutical composition for suppressing alcohol absorption in a patient with kidney disease;
(3-13) Use of citric acid or a salt thereof for producing a pharmaceutical composition for promoting alcohol metabolism;
(3-14) Use of citric acid or a salt thereof for producing a pharmaceutical composition for promoting alcohol metabolism in a patient with kidney disease;
(3-15) Use of citric acid or a salt thereof for producing a pharmaceutical composition for increasing a blood ornithine level, a blood serotonin level, a blood taurine level, a blood cystine level, or a blood nicotine amide level; and
(3-16) Use of citric acid or a salt thereof for producing a pharmaceutical composition for increasing a blood ornithine level, a blood serotonin level, a blood taurine level, a blood cystine level, or a blood nicotine amide level in a patient with kidney disease.

Hereinafter, the present invention will be further described with reference to Examples, but the present invention is not limited thereto.

### Examples

### Example 1

Four citric acid formulation tablets (each containing 231.5 mg of potassium citrate, 195.0 mg of sodium citrate hydrate, and 72.5 mg of citric acid) were mixed with 200 g of a forage, and miniature pig models with renal damage due to ligation of renal vessels (Gottingen Minipigs, male, n = 3, 20 months old, Oriental Yeast Co., Ltd., Ina MP Breeding Center) were fed with the resulting mixture three times per day for 7 days (stage I). Thereafter, a 7-day washout period was set, and the forage containing four citric acid formulation tablets was fed three times per day for 7 days (stage II). Blood samples were collected at 4 points: before administration of the citric acid formulation in stage I (1), the last day of administration (2), the last day of washout period (3), and the last day of administration in stage II (4), and plasma samples were obtained. After appropriate pretreatment, the samples were measured for metabolites by capillary electrophoresis-mass spectrometry using CE-TOFMS (Agilent Technologies, Inc.). The amount of the obtained metabolites was calculated as a relative area value, and comparison between groups was performed by Welch's t-test.

As a result, the following was confirmed.

It was confirmed that the ornithine level in stage II (4) was increased compared to that before administration of the citric acid formulation (1) (p = 0.0711) (Fig. 1).

It was confirmed that the serotonin level in stage II (4) was increased compared to that in the washout period (3) (p = 0.0511) (Fig. 2).

It was confirmed that the taurine level tended to be increased in stage I (2) compared to that before the start of the test (1), and the taurine level tended to be increased in stage II (4) compared to that in the washout period (3) (Fig. 3).

It was confirmed that the cystine level in stage II (4) was significantly increased compared to that in the washout period (3) (Fig. 4).

It was confirmed that the nicotinamide level in stage II (4) was increased compared to that before the start of the test (1) and that in the washout period (2) (Fig. 5).

### Example 2

A citrate solution (an aqueous solution containing 13.92 g of tripotassium citrate, 11.7 g of trisodium citrate, and 4.38 mg of anhydrous citric acid in 100 mL) was administered to SD male rats (8 weeks old) at a dose of 10 mL/kg. 30 minutes after administration, blood samples were collected, and then an ethanol solution (10 mL (20 v/w%)/kg) was administered (hereinafter, the target rats to which the citrate solution was administered may be referred to as a citrate group). After that, blood samples were collected every 30 minutes for up to 2 hours. The blood ethanol level was measured by the enzyme method, the blood acetaldehyde level was measured by the HPLC method, the blood acetic acid level was measured by the enzyme method, the blood lactic acid level was measured by the enzyme method, and the blood pyruvic acid level was measured by the enzyme method. As a control group, purified water was administered instead of the aqueous citrate solution. Further, the lactic acid/pyruvic acid ratio was determined from the lactic acid level and the pyruvic acid level at the same time of blood collection. The results are shown in Figs. 6 to 11.

As a result, regarding ethanol, lower values were observed in the citrate group as compared to the values in the control group at any time of 0.5 to 2 hours after administration. Acetaldehyde and acetic acid also showed almost the same tendency as ethanol. It was considered that the administration of citrate promoted ethanol metabolism and/or inhibited absorption.

In the citrate group, the blood alcohol level hardly increased immediately after alcohol ingestion. This suggests that the administration of the aqueous citrate solution suppresses alcohol absorption. Further, in the citrate group, pyruvic acid in blood and lactic acid in blood tend to increase 1 hour after administration of ethanol, and NAD⁺ produced by the conversion of pyruvic acid to lactic acid is considered to promote alcohol metabolism. This suggests that the administration of the aqueous citrate solution promotes alcohol metabolism.

Regarding lactic acid and pyruvic acid, higher values in the citrate group were shown, as compared to the values before administration at any time of 0.5 to 2 hours after administration. Meanwhile, regarding the lactic acid/pyruvic acid ratio, the lactic acid/pyruvic acid ratio in the control group was always higher than that in the citrate group, and showed an increasing tendency with the passage of time. The lactic acid/pyruvic acid ratio in the citrate group was changed within a certain range from the value (normal value) before administration of the ethanol solution. Therefore, it was confirmed that the abnormal blood lactic acid/pyruvic acid ratio was suppressed by the administration of citrate.

### Industrial Applicability

It is possible to provide a food composition or pharmaceutical composition useful in suppressing alcohol sickness or hangover due to alcoholic beverage ingestion; suppressing an increase in blood alcohol level, an increase in blood acetaldehyde level, or an increase in blood acetic acid level due to alcoholic beverage ingestion; suppressing a decrease in blood pyruvic acid level; suppressing an abnormal blood lactic acid/pyruvic acid ratio due to alcoholic beverage ingestion; suppressing an increase in blood alcohol level immediately after alcohol ingestion; suppressing alcohol absorption; promoting alcohol metabolism; or increasing a blood ornithine level, a blood serotonin level, a blood taurine level, a blood cystine level, or a blood nicotine amide level, in mammals, particularly humans.

## Claims

1. A food composition or pharmaceutical composition for suppressing alcohol sickness or hangover due to alcoholic beverage ingestion in mammals, comprising, as an active ingredient, citric acid or a salt thereof, wherein the food composition or pharmaceutical composition is a tablet.

2. A food composition or pharmaceutical composition for suppressing an increase in blood alcohol level, an increase in blood acetaldehyde level, or an increase in blood acetic acid level due to alcoholic beverage ingestion in mammals, comprising, as an active ingredient, citric acid or a salt thereof, wherein the food composition or pharmaceutical composition is a tablet.

3. A food composition or pharmaceutical composition for suppressing a decrease in blood pyruvic acid level due to alcoholic beverage ingestion in mammals, comprising, as an active ingredient, citric acid or a salt thereof, wherein the food composition or pharmaceutical composition is a tablet.

4. A food composition or pharmaceutical composition for suppressing an abnormal blood lactic acid/pyruvic acid ratio due to alcoholic beverage ingestion in mammals, comprising, as an active ingredient, citric acid or a salt thereof, wherein the food composition or pharmaceutical composition is a tablet.

5. A food composition or pharmaceutical composition for suppressing an increase in blood alcohol level immediately after alcohol ingestion in mammals, comprising, as an active ingredient, citric acid or a salt thereof, wherein the food composition or pharmaceutical composition is a tablet.

6. A food composition or pharmaceutical composition for suppressing alcohol absorption in mammals, comprising, as an active ingredient, citric acid or a salt thereof, wherein the food composition or pharmaceutical composition is a tablet.

7. A food composition or pharmaceutical composition for promoting alcohol metabolism in mammals, comprising, as an active ingredient, citric acid or a salt thereof, wherein the food composition or pharmaceutical composition is a tablet.

8. The food composition or pharmaceutical composition according to any one of claims 1 to 7, wherein the active ingredient is an alkali metal salt of citric acid.

9. The food composition or pharmaceutical composition according to any one of claims 1 to 8, wherein the alkali metal salt of citric acid includes a mixture of sodium citrate or a hydrate thereof and potassium citrate or a hydrate thereof.

10. The food composition or pharmaceutical composition according to any one of claims 1 to 9 comprising: sodium citrate or a hydrate thereof; potassium citrate or a hydrate thereof; and anhydrous citric acid.

11. The food composition or pharmaceutical composition according to any one of claims 1 to 10, wherein the food composition or pharmaceutical composition is a food composition whose package, container, or instruction indicates an effect of suppressing alcohol sickness or hangover due to alcoholic beverage ingestion; an effect of suppressing an increase in blood alcohol level, an increase in blood acetaldehyde level, or an increase in blood acetic acid level due to alcoholic beverage ingestion; an effect of suppressing a decrease in blood pyruvic acid level due to alcoholic beverage ingestion; an effect of suppressing an abnormal blood lactic acid/pyruvic acid ratio due to alcoholic beverage ingestion; an effect of suppressing an increase in blood alcohol level immediately after alcohol ingestion; an effect of suppressing alcohol absorption; and/or an effect of promoting alcohol metabolism.

12. A food composition or pharmaceutical composition for increasing a blood ornithine level, a blood serotonin level, a blood taurine level, a blood cystine level, or a blood nicotine amide level in mammals, comprising, as an active ingredient, citric acid or a salt thereof, wherein the food composition or pharmaceutical composition is a tablet.

13. The food composition or pharmaceutical composition according to claim 12, wherein the active ingredient is an alkali metal salt of citric acid.

14. The food composition or pharmaceutical composition according to any one of claims 1 to 13, wherein the active ingredient is anhydrous citric acid, sodium citrate or a hydrate thereof, and potassium citrate or a hydrate thereof, a molar ratio of the anhydrous citric acid: the sodium citrate or the hydrate thereof: the potassium citrate or the hydrate thereof is in a range of 1 : 1.7-2.3 : 1.7-2.3, and the food composition or pharmaceutical composition is administered or ingested before alcohol ingestion.
